# EUROPEAN PATENT APPLICATION

(11) **EP 4 697 010 A1**
(43) Date of publication of application: **18.02.2026**
(21) Application number: 24841413.8
(22) Date of filing: 01.08.2024
(51) Int. Cl.: G01N 15/14

(54) **REAL-TIME QUANTITATIVE DETECTION METHOD FOR NEXT-GENERATION PROBIOTICS ON BASIS OF FLOW CYTOMETRY AND USE THEREOF**

(30) Priority: 19.06.2024 CN 202410793007
(71) Applicant: Wecare Probiotics Co., Ltd., Suzhou, Jiangsu 215200 (CN)
(72) Inventor: FANG, Shuguang, Suzhou, Jiangsu 215200 (CN); YAN, Qifang, Suzhou, Jiangsu 215200 (CN); JIANG, Dacheng, Suzhou, Jiangsu 215200 (CN); ZHOU, Heyu, Suzhou, Jiangsu 215200 (CN); CHEN, Yemin, Suzhou, Jiangsu 215200 (CN)
(74) Representative: Buzzi, Notaro & Antonielli d'Oulx S.p.A.
(86) International application number: PCT/CN2024/109107
(87) International publication number: WO 2025/260461

(57) **Abstract**

Provided are a real-time quantitative detection method for next-generation probiotics based on flow cytometry and use thereof. The next-generation probiotics includes *Akkermansia muciniphila* and/or *Bacteroides fragilis.* In the present application, the flow cytometry is applied to the detection of next-generation probiotics, which can provide a quantitative data of viable cells and dead cells in real time, quickly complete the high-throughput detection of the viable cell count in a sample, reduce the risk of pollution in the detection process, and also detect the next-generation probiotics that cannot be conventionally cultured due to insufficient metabolic vitality to be cultured; and the detection speed is fast and the accuracy rate is high.

## Description

### TECHNICAL FIELD

The present application belongs to the technical field of microbiology, and specifically relates to a real-time quantitative detection method for next-generation probiotics based on flow cytometry and use thereof.

### BACKGROUND

Intestinal flora is a microbial flora that lives in the human intestines. The number of bacteria parasitic in the human intestines is 40 trillion, and the total number of genes is about 150 times that of human own genes. It can be seen that the intestinal flora is a very large group, which is also known as the "second genome", "second brain", or "intestinal brain" of the human body. Under normal circumstances, the intestinal flora can establish a dynamic ecological balance with the host and the external environment. Once the intestinal flora is out of balance, the host will suffer from loss of barrier function, inflammation and loss of immune function, thus inducing diseases in the host.

Next-generation probiotics (NGPs) is an intestinal microflora that can intervene with human diseases. Next-generation probiotics refer to living microorganisms that have not been determined yet, but when given enough doses, they can have an impact on animal health and production characteristics through specific actions, and are suitable for preventing and treating metabolic disorders or improving host health. NGPs take intestinal symbiotic bacteria as a main body, and are made into microecological preparations by artificial design, which act on the host to reshape intestinal microorganisms to play specific functions. At present, the known next-generation probiotics include: *Akkermansia muciniphila* and *Bacteroides fragilis.*

*Akkermansia muciniphila* (Akk bacterium) is a common human intestinal symbiotic bacteria, which can survive by mucin in the intestinal mucus layer,, mainly colonize the outer mucus layer of the gastrointestinal tract, and take mucin in the gastrointestinal tract as the source of carbon and nitrogen for its own growth. Mucin consumed by *Akkermansia muciniphila* and mucin regenerated from goblet cells can achieve dynamic balance, thus maintaining the stability of mucus layer. In the past decade, more and more studies have demonstrated that patients with diabetes, cardiovascular disease, diseased intestinal diseases and neurologic diseases have a lower abundance of Akk bacterium in the gastrointestinal tract, which also indicates that Akk bacterium will be the next generation probiotics with promising clinical applications, especially in the prevention and treatment of diabetes, obesity, and cancer.

*Bacteroides fragilis* is a symbiotic bacterium colonized in the intestinal tract of mammals, which is essential to maintain the normal function of the host, and the beneficial effect of PSA secreted by Bacteroides fragilis has become a consensus, so Bacteroides fragilis has a good development prospect as a potential probiotic strain. Several studies have further revealed that *Bacteroides fragilis* is closely associated with inflammatory enteritis and immune disease. At the same time, compared with other anaerobic bacteria in gastrointestinal tract, *Bacteroides fragilis* showed the highest antibiotic resistance and the most antibiotic resistance mechanism, which not only makes it difficult to treat infections caused by *Bacteroides fragilis,* but also has the potential to act become a reservoir for antibiotic resistance genes, leading to their transfer to other normal bacterial flora through the integration of transposons and the integration of genetic elements, so that they can be transferred to other normal bacterial flora through the integration of transposons and the integration of genetic elements.

In 2022, Chinese Institute of Food Science and Technology published "T/CIFST 009-2022 General Standard of Probiotics for Food Use", which made basic regulations for the basic requirements, bacterial strain level requirements, production process requirements, technical requirements, storage and transportation, and applications in food, and label identification of probiotics used as food raw materials, as well as the requirements for the probiotic viable bacterial count ≥ 1.0 × 10⁸ CFU/ g (mL), where CFU is a colony forming unit, and refers to a colony formed by the growth and reproduction of a single bacterium or a group of multiple bacteria on the solid medium in the bacterial culture, which is called the colony forming unit to show the number of viable bacteria.

Currently, the detection methods for next-generation probiotics are scarce. In the conventional culture counting, some damaged cells have insufficient metabolic vitality so that they cannot be counted, and the accuracy needs to be improved; moreover, some next-generation probiotics have high requirements for the culture environment, and the detection results are lagging behind.

In summary, how to develop an efficient and accurate detection method for next-generation probiotics and apply it to real-time monitoring of the processing and production of next-generation probiotics has become an urgent problem in this field.

### SUMMARY

The present application provides a real-time quantitative detection method for next-generation probiotics based on flow cytometry and use thereof. In the present application, the next-generation probiotics are pre-treated and stained, and detected by instrument for 1 min to obtain the data of viable bacteria count and a proportion of cell state, which provides the viable bacteria quantitative detection data and quality evaluation in real time for rapid analysis in the production process.

In a first aspect, the present application provides a real-time quantitative detection method for next-generation probiotics based on flow cytometry, wherein the next-generation probiotics includes *Akkermansia muciniphila* and/or *Bacteroides fragilis.* Preferably, the real-time quantitative detection method for next-generation probiotics based on flow cytometry includes: pre-treating and staining next-generation probiotics to obtain a sample to be detected, placing the sample to be detected into a flow cytometer for detecting to obtain a detection result, and analyzing the data of the detection result.

Preferably, the pre-treating includes: mixing the next-generation probiotics with a diluent to obtain a diluted next-generation probiotic.

Preferably, the diluent includes peptone and an inorganic salt.

The diluent provided in the present application can be used to adjust a concentration of the sample to be detected and has a protective effect on bacteria, which is suitable for detecting the next-generation probiotics by flow cytometry, and especially suitable for detecting anaerobe and/or facultative anaerobe in the next-generation probiotics.

Preferably, the peptone includes any one or a combination of at least two of casein peptone, yeast peptone, or soy peptone.

Preferably, the inorganic salt includes any one or a combination of at least two of sodium chloride, sodium bicarbonate, disodium hydrogen phosphate, or potassium dihydrogen phosphate.

Preferably, in the diluent, the peptone has a concentration of 0.8-1.2 g/L (for example, 0.8 g/L, 0.9 g/L, 1.0 g/L, 1.1 g/L, or 1.2 g/L, etc.), and the inorganic salt has a concentration of 7-10 g/L (for example, 7 g/L, 8 g/L, 8.5 g/L, 9 g/L, or 10 g/L, etc.).

Preferably, the diluent includes 1.0 g/L of casein peptone and 8.5 g/L of sodium chloride.

Preferably, the staining includes: mixing the diluted next-generation probiotics having a particle number of 2×10³-2×10⁴/µL (for example, 2×10³/µL, 4×10³/µL, 6×10³/µL, 8×10³/µL, 1×10⁴/µL, 1.2×10⁴/µL, 1.4×10⁴/µL, 1.6×10⁴/µL, 1.8×10⁴/µL, or 2×10⁴/µL, etc.) with a dye, performing vortex-mixing, and then incubating for 10-20 min (for example, 10 min, 13 min, 15 min, 17 min, or 20 min, etc.) in the dark.

Preferably, the dye includes any one or a combination of at least two of SYTO 9, SYTO 11, SYTO 16, or propidium iodide.

Preferably, in the dye, a concentration ratio of SYTO 9 to propidium iodide is 1: (0.8-1.2) (for example, 1:0.8, 1:0.9, 1:1, 1:1.1, or 1:1.2, etc.).

Preferably, a volume ratio of the dye to the diluted next-generation probiotics is 1: (800-1200) (for example, 1:800, 1:900, 1:1000, 1:1100, or 1:1200, etc.).

Too much or too little amount of the dye will lead to a great difference between parallel samples, and the volume ratio of the dye to the diluted next-generation probiotics is preferably 1:1000.

Preferably, the real-time quantitative detection method for next-generation probiotics based on flow cytometry further includes a step of making a template for data analysis.

Preferably, the template for data analysis includes a viable cell signal template and a dead cell signal template.

Preferably, a method for making a viable cell signal template includes: subjecting the next-generation probiotics to an activation treatment, and then immediately performing single-staining with SYTO 9 and placing on the flow cytometer for detecting, adjusting the B525-A channel voltage until the fluorescence signal is concentrated in the center or near the center area of the flow cytometry graph with FSC as the X-axis and SSC as the Y-axis, and obtaining a first voltage parameter and a first plot linear coefficient.

Preferably, a method for making a dead cell signal template includes: disrupting the cell membrane of the next-generation probiotics with isopropanol, and then performing double-staining with SYTO 9 and propidium iodide and placing on the flow cytometer for detecting, adjusting the B585-A channel voltage until the fluorescence signal is concentrated in the center or near the center area of flow cytometry graph with FSC as the X-axis and SSC as the Y-axis, and obtaining a second voltage parameter and a second plot linear coefficient.

Preferably, when the SYTO 9 and propidium iodide is used for double-staining, a concentration ratio of SYTO 9 to propidium iodide is 1: (0.8-1.2) (for example, 1:0.8, 1:0.9, 1:1, 1:1.1, or 1:1.2, etc.).

Preferably, placing the sample to be detected into the flow cytometer for detecting includes: exciting with a 488 nm argon ion laser equipped on the flow cytometer, and detecting the sample on the flow cytometer at a low speed; receiving the SYTO 9 fluorescence signal with the B525-A channel, adjusting the channel voltage to a first voltage parameter, and adjusting the plot to a first plot linear coefficient; receiving the propidium iodide fluorescence signal with the B585-A channel, adjusting the channel voltage to a second voltage parameter, and adjusting the plot to a second plot linear coefficient.

In the present application, the sample is detected on the flow cytometer at a low speed mode, and the standard deviation of the three repetitions is smaller and the detection stability is higher.

Preferably, the data analysis includes: adjusting a B525-A channel compensation and a B585-A channel compensation to separate the corresponding negative group and the corresponding positive group, correcting the blank fluorescent background, and comparing with the viable cell signal template and the dead cell signal template respectively, determining a location of the viable cell group and the dead cell group, then gating, and calculating the viable bacteria count based on the particle number shown on the instrument AFU=N×a×1000, wherein N is a result of a viable bacteria concentration detected by the instrument (particle number/µL), and a is a dilution multiple of the sample.

The final result is reported in the form of rounding off the third significant figure and retaining the first two significant figures while replacing the other figures with zero or expressing them in the form of an exponent.

Preferably, the real-time quantitative detection method for next-generation probiotics based on flow cytometry includes the following steps:
(1) mixing the next-generation probiotics with a diluent to obtain a diluted next-generation probiotics, wherein the diluent includes peptone and an inorganic salt;
(2) mixing the diluted next-generation probiotics in step (1) with a dye to obtain a sample to be detected, wherein the dye includes SYTO 9 and propidium iodide;
(3) placing the sample to be detected in step (2) into a flow cytometer for detecting, and receiving a fluorescence signal of SYTO 9 and propidium iodide to obtain a detection result; and
(4) calculating a viable bacteria count and a proportion of dead and viable bacteria in the next-generation probiotics based on the detection result in step (3).

Preferably, the real-time quantitative detection method for next-generation probiotics based on flow cytometry is performed in an anaerobic condition.

In a second aspect, the present application provides a real-time quantitative detection instrument for next-generation probiotics. The real-time quantitative detection instrument is used for performing steps in the real-time quantitative detection method for next-generation probiotics based on flow cytometry according to the first aspect.

Preferably, the real-time quantitative detection instrument includes a sample detection unit and a data analysis unit.

Preferably, the sample detection unit is used for performing the following steps: pre-treating and staining the next-generation probiotics to obtain a sample to be detected, and placing the sample to be detected to a flow cytometer for detecting to obtain a detection result.

Preferably, the data analysis unit is used for performing data analysis on the detection result.

In a third aspect, the present application provides a use of the real-time quantitative detection method for next-generation probiotics based on flow cytometry according to the first aspect and/or the real-time quantitative detection instrument for next-generation probiotics according to the second aspect in the next-generation probiotics product development and/or the production process development of the next-generation probiotic.

In the present application, the analysis of the proportion of viable cells and dead cells in next-generation probiotics can be applied to the fermentation process and the stability tracking of the product during the shelf-life, as well as the evaluation of the culture purity, which can provide the data support for the parameter control of the fermentation process and the quality control of the product, and it is of great significance for the industrialization of the next-generation probiotics.

Other specific point values within the above value ranges are optional and will not be repeated here.

Compared to the prior art, the present application has the following beneficial effects.

In the present application, the flow cytometry is applied to the detection of next-generation probiotics, which can provide a quantitative data of viable cells and dead cells in real time, quickly complete the high-throughput detection of the viable cell count in a sample, reduce the risk of pollution in the detection process, and also detect the next-generation probiotics that cannot be conventionally cultured due to insufficient metabolic vitality to be cultured; and the detection speed is fast and the accuracy is high. Most of the next-generation probiotics are anaerobic bacteria, the conventional culture and detection process takes a long time and requires a high degree of anaerobic. In the present application, an anaerobic workstation can be used to provide an anaerobic environment in the sample treatment process, reducing the loss of viable bacteria counts caused by the environment during sample treatment. In the present application, the proportion analysis of viable cells and dead cells in next-generation probiotics can be applied to the fermentation process and the stability tracking of the product during the shelf-life, which is of great significance for the industrialization of the next-generation probiotics.

### BRIEF DESCRIPTION OF DRAWINGS

FIG. 1 is a graph showing the detection results of the blank samples by flow cytometry in Example 1.
FIG. 2 is a template diagram showing dead cell signals by flow cytometry in Example 1.
FIG. 3 is a graph showing the detection results of flow cytometry in Example 1.
FIG. 4 is a graph showing the detection result of flow cytometry in Example 11.
FIG. 5 is a graph showing the detection result of flow cytometry in Example 12.

### DETAILED DESCRIPTION

In order to further illustrate the technical methods adopted in the present application and the effect thereof, the present application is further described below in conjunction with embodiments and drawings. It can be understood that the embodiments described here are only used to explain the present application, but not to limit the present application.

In examples, those without indicating specific technology or conditions are in accordance with the technology or conditions described in the literature of the field, or in accordance with the product specification. The reagents or instruments used without indicating the manufacturer are conventional products available commercially through regular means.

Strains, reagents and instruments used in the following examples:
Strains involved in the following examples:
*Akkermansia muciniphila* Akk11, which is deposited in China Center for Type Culture Collection on Jan. 15, 2024, with the deposit number of CCTCC NO. M2024119, and the address of Wuhan University, No. 299, Bayi Road, Wuchang District, Wuhan City, Hubei Province, China.
*Bacteroides fragilis*: ATCC 25285, which is purchased from Guangdong Microbial Culture Collection Center.
Casein peptone, which is purchased from Qingdao Hopebio, and product number is HB8271.
SYTO 9 dye and PI dye, which are from LIVE/DEAD BacLight Bacterial Viability Kits, purchased from ThermoFisher, and a product number is L7012.
Flow cytometer, which is purchased from Beckman, and a product model is B53013.

### Example 1

This example provides a real-time quantitative detection method for *Akkermansia muciniphila* based on flow cytometry, which includes the following steps.

### (1) Reagent preparation

Diluent: 1.0 g/L of casein peptone and 8.5 g/L of sodium chloride were subjected to a moist-heat sterilization treatment at 121°C for 15 min, and then sub-packaged for use.

Mixed dye: in an anaerobic workstation, 1 µL of SYTO 9 dye and 1 µL of PI dye were sucked into a 1.5 mL centrifuge tube, subjected to vortex-mixing evenly, and then subjected to instantaneous centrifugation for later use.

### (2) Sample pretreatment

In an anaerobic workstation, 1 g of *Akkermansia muciniphila* powder was weighed out, added to 99 mL of diluent, mixed well and diluted.

### (3) Sample staining

A 10⁻⁴ gradient sample homogenate was sucked and added into the prepared mixed dye, wherein a volume ratio of the mixed dye to the sample was 1:1000, and subjected to vortex-mixing, then instantaneous centrifugation, and incubated for 15 min in the dark.

### (4) Instrument calibration

During the sample incubation process, the instrument was turned on and cleaned, and after the process, 2 mL of deionized water was taken for detecting by the instrument to confirm that the channel signal background was normal, and if the background signal was too strong, then continued to be cleaned with deionized water.

### (5) Preparation of blank control sample

The sterilized diluent was selected to be stained in the equal proportion for detecting by the instrument, and used as a reagent background blank.

### (6) Making a template for data analysis

A method of making a viable cell signal template includes: *Akkermansia muciniphila* was subjected to an activation treatment, and then subjected to single-staining with SYTO 9 immediately, and placed on the instrument for detecting, the B525-A channel voltage was adjusted until the fluorescence signal was concentrated in the center or near the center area of the flow cytometry graph with FSC as the X-axis and SSC as the Y-axis, and a first voltage parameter and a first plot linear coefficient were obtained.

A method of making a dead cell signal template includes: the cell membrane of the next-generation probiotics was disrupted with isopropanol, and then subjected to double-staining with SYTO 9 and propidium iodide, and placed on the instrument for detecting, the B585-A channel voltage was adjusted until the fluorescence signal was concentrated in the center or near the center area of the flow cytometry graph with FSC as the X-axis and SSC as the Y-axis, and a second voltage parameter and a second plot linear coefficient were obtained.

### (7) Sample detected on the instrument

The incubated samples were detected on the flow cytometer, and excited with a 488 nm argon ion laser equipped on the flow cytometer, the channel voltage was adjusted to the template voltage, the plot was adjusted to the template linear coefficient, the SYTO 9 fluorescence signal was received with the B525-A channel, and the PI fluorescence signal was received with the B585-A channel; the sample was detected on the instrument at a low speed, and each sample was repeated for three times.

### (8) Data analysis

The B525-A channel compensation and B585-A channel compensation were adjusted to separate the corresponding the negative group and positive group, the blank fluorescent background was corrected, then compared with the viable cell signal template and the dead cell signal template, respectively, and gated appropriately The viable bacteria count was calculated based on the number of particles displayed by the instrument: (AFU)=N×a×1000, wherein N was a concentration of the viable bacteria detected by the instrument (number of particles /µL), a is a dilution concentration of the sample, and 1000 is a volume unit conversion factor. The final result was reported in the form that the first two significant figures were retained after rounding the third significant figure, and other figures were replaced by zero or expressed in exponential form.

### Example 2

This example provides a real-time quantitative detection method for *Akkermansia muciniphila* based on flow cytometry, which differs from Example 1 only in that in step (7), a medium speed was selected for detecting samples on the instrument.

### Example 3

This example provides a real-time quantitative detection method for *Akkermansia muciniphila* based on flow cytometry, which differs from Example 1 only in that in step (7), a high speed was selected for detecting samples on the instrument.

### Example 4

This example provides a real-time quantitative detection method for *Akkermansia muciniphila* based on flow cytometry, which differs from Example 1 only in that in step (3), a volume ratio of the mixed dye to the sample was 1:700.

### Example 5

This example provides a real-time quantitative detection method for *Akkermansia muciniphila* based on flow cytometry, which differs from Example 1 only in that in step (3), a volume ratio of the mixed dye to the sample was 1:1300.

### Example 6

This example provides a real-time quantitative detection method for *Akkermansia muciniphila* based on flow cytometry, which differs from Example 1 only in that casein peptone in the diluent was replaced by soy peptone with an equal amount.

### Example 7

This example provides a real-time quantitative detection method for *Akkermansia muciniphila* based on flow cytometry, which differs from Example 1 only in that casein peptone in the diluent was replaced by yeast peptone with an equal amount.

### Example 8

This example provides a real-time quantitative detection method for *Akkermansia muciniphila* based on flow cytometry, which differs from Example 1 only in that the diluent was composed of 1.0 g/L of casein peptone, 8.5 g/L of sodium chloride, and 1.0 g/L of trehalose.

### Example 9

This example provides a real-time quantitative detection method for *Akkermansia muciniphila* based on flow cytometry, which differs from Example 1 only in that the diluent was composed of 1.0 g/L of casein peptone, 8.5 g/L of sodium chloride, and 1.0 g/L of saccharose.

### Example 10

This example provides a real-time quantitative detection method for *Akkermansia muciniphila* based on flow cytometry, which differs from Example 1 only in that the diluent was composed of 1.0 g/L of casein peptone, 8.5 g/L of sodium chloride, and 1.0 g/L of monosodium glutamate.

### Example 11

This example provides a real-time quantitative detection method for *Akkermansia muciniphila* based on flow cytometry, which differs from Example 1 only in that the *Akkermansia muciniphila* powder was stored in an incubator at 37°C for 4 weeks and then taken out.

### Example 12

This example provides a real-time quantitative detection method for *Akkermansia muciniphila* based on flow cytometry, which differs from Example 1 only in that the detected sample was *Bacteroides fragilis.*

### Comparative Example 1

This comparative example provides a method for detecting the viable bacteria count of *Akkermansia muciniphila* by a culture counting method, which includes the following steps.
(1) Preparation of culture medium
   2 g/L of mucin and 10 g/L of agar powder were added to commercial brain-heart infusion broth to obtain an *Akkermansia muciniphila* culture medium.
(2) Culture and result statistics

The samples were diluted in the anaerobic workstation, and 1 mL of 10⁻⁸ sample homogenate was sucked into a sterile petri dish, poured with 15 mL of culture medium, and cultured anaerobically at 37°C for 96 h. After the culture, the samples were counted manually.

### Test Example 1

In this test example, the methods provided in Examples 1-10 were used to detectthe viable bacteria count of the same *Akkermansia muciniphila,* and three parallel experiments were conducted in each group, and the average and standard deviation of the results of the three experiments were calculated. The results are shown in Table 1.

**Table 1**

| | Testing result (hundred million AFU/g) | | | Average value (hundred million AFU/g) | Standard deviation |
|---|---|---|---|---|---|
| Example 1 | 440 | 440 | 440 | 440 | 33.62 |
| Example 2 | 440 | 430 | 440 | 430 | 49.02 |
| Example 3 | 430 | 420 | 440 | 430 | 72.71 |
| Example 4 | 430 | 420 | 410 | 420 | 92.54 |
| Example 5 | 390 | 410 | 400 | 400 | 118.33 |
| Example 6 | 390 | 410 | 410 | 410 | 126.58 |
| Example 7 | 420 | 420 | 430 | 420 | 75.12 |
| Example 8 | 400 | 390 | 410 | 400 | 108.42 |
| Example 9 | 400 | 380 | 410 | 390 | 123.15 |
| Example 10 | 420 | 400 | 410 | 410 | 115.8 |

The following can be concluded from Table 1.
(1) Comparing Example 1 with Examples 2 and 3, it can be seen that in Examples 2 and 3, samples were detected at medium speed and high speed respectively, and the standard deviation of three repetitions was greater than that in Example 1, therefore, in order to improve the detection stability, it is preferred to detect samples at a low speed.
(2) Comparing Example 1 with Examples 4 and 5, it can be seen that too much or too little amount of the dye can lead to great differences among parallel samples.
(3) Comparing Example 1 with Examples 6 and 7, it can be seen that the protective effect of casein peptone on bacteria is the best, and the standard deviation of detection results is the smallest.
(4) Comparing Example 1 with Examples 8-10, it can be seen that adding other components to the original diluent composed of casein peptone and sodium chloride can reduce the stability of the detection method.

### Test Example 2

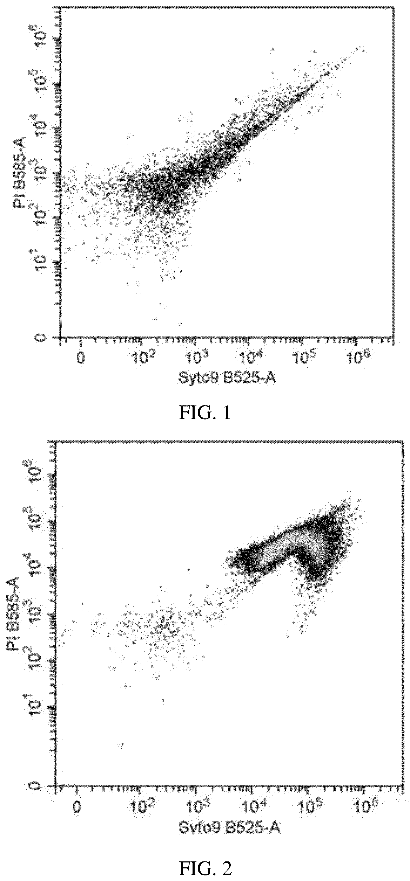

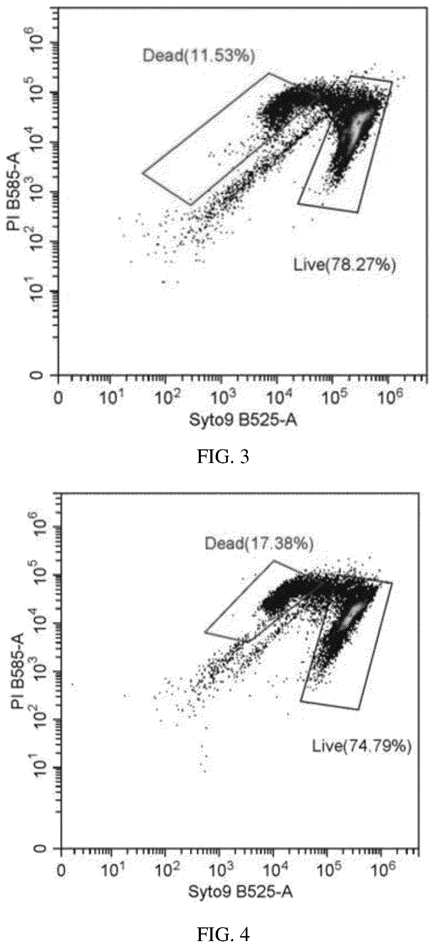

In this test example, the methods provided in Example 1 and Example 11 were used to detect the viable bacteria count of the same *Akkermansia muciniphila.* The detection results of the blank sample in Example 1 are shown in FIG. 1, the dead cell signal template in FIG. 2, the detection results in Example 1 in FIG. 3 and the detection results in Example 11 in FIG. 4.

As can be seen from FIG. 4, the samples after heating treatment shows the cluster of dead bacterial cells in the dead area, which is not obviously different from the location and shape of the cell cluster showed in the dead bacteria template of FIG. 2, and the proportion have a corresponding variation trend, indicating that this method can effectively distinguish between the viable bacteria count and the dead bacterial count in the sample, and can provide a viable-dead proportion.

### Test Example 3

In this test example, the methods provided in Example 1 and Comparative Example 1 were used to detect the viable bacteria count of the same *Akkermansia muciniphila,* and three parallel experiments were conducted in each group. The results are shown in Table 2.

**Table 2**

| | Detection result (hundred million AFU/g) | | | Average value (hundred million AFU/g) |
|---|---|---|---|---|
| Example 1 | 440 | 440 | 440 | 440 |
| Comparative Example 1 | 360 | 360 | 350 | 360 |

Comparing Example 1 with Comparative Example 1, it can be seen that the conventional culture method of Comparative Example 1 takes an average of 97 h for each sample from sample processing to result issuance, and the amount of reagents preparation in the early stage is large, and the cleaning time of consumables in the later stage is long, which consumes labor, and the saturated workload of one detector is 12 samples/day; whereas, the method provided in the present application can save half the time, reduce the preparation amount of related reagents by half, and get the data results within 1 min after a single sample is detected on the instrument.

It can be seen from the detection result that the results of the conventional culture method are lower than those calculated by flow cytometer. The reason is that some cells that cannot be cultured due to insufficient metabolic viability cannot be detected by the conventional culture method. In addition, the detection result of the culture method are manually counted and calculated, and the data requires to be input manually for processing during the result analysis, while the real-time quantitative detection method is adopted, and the data can be directly exported in multiple batches.

### Test Example 4

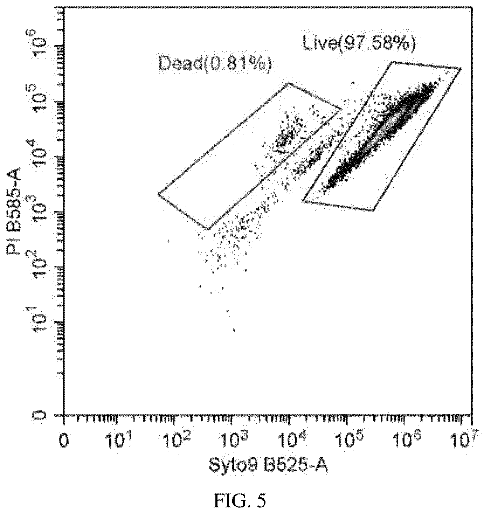

In this test example, the method provided in Example 12 was used to detect the viable bacteria count of the same *Bacteroides fragilis.* The results are shown in FIG. 5.

It is calculated from FIG. 5 that *Bacteroides fragilis* has an amount of viable bacteria particles of 2379.78 and an amount of dead bacterial particles of 593.15, and the viable bacteria count is 2.4×108 AFU/g.

In summary, the present application provides a real-time quantitative detection method for next-generation probiotics based on flow cytometry and use thereof, which provides quantitative data of viable cells and dead cells in real time, and greatly improves the accuracy and the detection speed compared with the conventional culture method. The detection method is especially suitable for the real-time quantitative detection of anaerobic bacteria, and can be widely applied to the industrial development of next-generation probiotics.

The applicant declares that the above is specific embodiments of the present application, but the protective scope of the present application is not limited thereto. Those skilled in the art should understand that any variation or substitution, which can be easily thought of by those skilled in the art within the technical scope disclosed in the present application, shall fall within the protection scope and disclosure scope of the present application.

## Claims

1. A real-time quantitative detection method for next-generation probiotics based on flow cytometry, wherein the next-generation probiotics comprise *Akkermansia muciniphila* and/or *Bacteroides fragilis.*

2. The real-time quantitative detection method for next-generation probiotics based on flow cytometry according to claim 1, wherein the real-time quantitative detection method for next-generation probiotics based on flow cytometry comprises: pre-treating and staining next-generation probiotics to obtain a sample to be detected, placing the sample to be detected into a flow cytometer for detecting to obtain a detection result, and analyzing the data of the detection result.

3. The real-time quantitative detection method for next-generation probiotics based on flow cytometry according to claim 2, wherein the pre-treatment comprises: mixing the next-generation probiotics with a diluent to obtain a diluted next-generation probiotic;
preferably, the diluent comprises peptone and an inorganic salt;
preferably, the peptone comprises any one or a combination of at least two of casein peptone, yeast peptone, or soy peptone;
preferably, the inorganic salt comprises any one or a combination of at least two of sodium chloride, sodium bicarbonate, disodium hydrogen phosphate, or potassium dihydrogen phosphate;
preferably, in the diluent, the peptone has a concentration of 0.8-1.2 g/L, and the inorganic salt has a concentration of 7-10 g/L.

4. The real-time quantitative detection method for next-generation probiotics based on flow cytometry according to claim 2, wherein the staining comprises: mixing the diluted next-generation probiotics having a particle number of 10³-2×10⁴/µL with a dye, performing vortex-mixing, and then incubating for 10-20 min in the dark;
preferably, the dye comprises any one or a combination of at least two of SYTO 9, SYTO 11, SYTO 16, or propidium iodide;
preferably, in the dye, a concentration ratio of SYTO 9 to propidium iodide is 1: (0.8-1.2);
preferably, a volume ratio of the dye to the diluted next-generation probiotics is 1: (800-1200).

5. The real-time quantitative detection method for next-generation probiotics based on flow cytometry according to claim 2, wherein the real-time quantitative detection method for next-generation probiotics based on flow cytometry further comprises a step of making a template for data analysis;
preferably, the template for data analysis comprises a viable cell signal template and a dead cell signal template;
preferably, a method for making a viable cell signal template comprises: subjecting the next-generation probiotics to an activation treatment, and then immediately performing single-staining with SYTO 9 and placing on the flow cytometer for detecting, adjusting the B525-A channel voltage until the fluorescence signal is concentrated in the center or near the center area of the flow cytometry graph with FSC as the X-axis and SSC as the Y-axis, and obtaining a first voltage parameter and a first plot linear coefficient;
preferably, a method for making a dead cell signal template comprises: disrupting the cell membrane of the next-generation probiotics with isopropanol, and then performing double-staining with SYTO 9 and propidium iodide, and placing on the flow cytometer for detecting, adjusting the B585-A channel voltage until the fluorescence signal is concentrated in the center or near the center area of the flow cytometry graph with FSC as the X-axis and SSC as the Y-axis, and obtaining a second voltage parameter and a second plot linear coefficient.

6. The real-time quantitative detection method for next-generation probiotics based on flow cytometry according to claim 2, wherein placing the sample to be detected into the flow cytometer for detecting comprises: exciting with a 488 nm argon ion laser equipped on the flow cytometer and detecting the sample on the flow cytometer at a low speed; receiving a SYTO 9 fluorescence signal with the B525-A channel, adjusting a channel voltage to a first voltage parameter, and adjusting the plot to a first plot linear coefficient; receiving a propidium iodide fluorescence signal with the B585-A channel, adjusting a channel voltage to a second voltage parameter, and adjusting the plot to a second plot linear coefficient.

7. The real-time quantitative detection method for next-generation probiotics based on flow cytometry according to claim 2, wherein the data analysis comprises: adjusting a B525-A channel compensation and a B585-A channel compensation to separate the corresponding a negative group and a positive group, correcting a blank fluorescent background, and comparing with the viable cell signal template and the dead cell signal template respectively, determining a location of a viable cell group and a dead cell group, then gating, and calculating a viable bacteria count based on the particle number shown on the instrument AFU=N×a×1000, wherein N is a result of a viable bacteria concentration detected by the instrument, with a unit of particle number/µL, and a is a dilution multiple of the sample.

8. The real-time quantitative detection method for next-generation probiotics based on flow cytometry according to any one of claims 1-7, wherein the real-time quantitative detection method for next-generation probiotics based on flow cytometry comprises the following steps:
(1) mixing the next-generation probiotics with a diluent to obtain a diluted next-generation probiotics, wherein the diluent comprises peptone and an inorganic salt;
(2) mixing the diluted next-generation probiotics in step (1) with a dye to obtain a sample to be detected, wherein the dye comprises SYTO 9 and propidium iodide;
(3) placing the sample to be detected in step (2) into a flow cytometer for detecting, and receiving a fluorescence signal of SYTO 9 and propidium iodide to obtain a detection result; and
(4) calculating a viable bacteria count and a proportion of dead and viable bacteria in next-generation probiotics based on the detection result in step (3).

9. A real-time quantitative detection instrument for next-generation probiotics, which is used for performing steps in the real-time quantitative detection method for next-generation probiotics based on flow cytometry according to any one of claims 1-8;
preferably, the real-time quantitative detection instrument comprises a sample detection unit and a data analysis unit;
preferably, the sample detection unit is used for performing the following steps: pre-treating and staining the next-generation probiotics to obtain a sample to be detected, and placing the sample to be detected into a flow cytometer for detecting to obtain a detection result;
preferably, the data analysis unit is used for performing data analysis on the detection result.

10. Use of the real-time quantitative detection method for next-generation probiotics based on flow cytometry according to any one of claims 1-8 and/or the real-time quantitative detection instrument for next-generation probiotics according to claim 9 in the next-generation probiotics product development and/or the next-generation probiotics production process development.
